# EUROPEAN PATENT SPECIFICATION

(11) **EP 4 351 516 B1**
(45) Date of publication and mention of the grant of the patent: **25.11.2026**
(21) Application number: 22819775.2
(22) Date of filing: 31.05.2022
(51) Int. Cl.: A61L 15/28, A61F 13/01, A61L 15/18, A61L 15/32, A61L 15/42, C08L 5/08, C08L 89/00, A61F 13/00

(54) **LAYERED COMPOSITION AND IMPLEMENTATIONS THEREOF**
GESCHICHTETE ZUSAMMENSETZUNG UND IMPLEMENTIERUNGEN DAVON
COMPOSITION EN COUCHES ET SES MISES EN OEUVRE

(30) Priority: 09.06.2021 IN 202141025757
(43) Date of publication of application: 17.04.2024
(73) Proprietor: Indian Institute of Science, Bangalore Karnataka 560012 (IN); Fibroheal Woundcare Pvt. Ltd., Karnataka Bengaluru 560064 (IN)
(72) Inventor: INDRAKUMAR, Sushma, Karnataka Bengaluru 560012 (IN); GHOSH, Santanu, Karnataka Bengaluru 560012 (IN); CHATTERJEE, Kaushik, Karnataka Bengaluru 560012 (IN); DASH, Tapan Kumar, Karnataka Bengaluru 560064 (IN); MISHRA, Vivek, Karnataka Bengaluru 560064 (IN); TANDON, Bharat, Karnataka Bengaluru 560064 (IN)
(74) Representative: Lavoix
(86) International application number: PCT/IN2022/050506
(87) International publication number: WO 2022/259263

(56) References cited:
- WO-A1-2020/247594
- CN-A- 103 418 022
- CN-A- 109 260 507
- CN-B- 102 772 820
- US-B2- 9 433 698
- BELDA MARÍN CRISTINA, FITZPATRICK VINCENT, KAPLAN DAVID L., LANDOULSI JESSEM, GUÉNIN ERWANN, EGLES CHRISTOPHE: "Silk Polymers and Nanoparticles: A Powerful Combination for the Design of Versatile Biomaterials", FRONTIERS IN CHEMISTRY, vol. 8, 1 December 2020 (2020-12-01), XP093014184, DOI: 10.3389/fchem.2020.604398
- BARROSO ANDREIA, MESTRE HENRIQUE, ASCENSO ANDREIA, SIMÕES SANDRA, REIS CATARINA: "Nanomaterials in wound healing: From material sciences to wound healing applications", NANO SELECT, WILEY, vol. 1, no. 5, 1 November 2020 (2020-11-01), pages 443 - 460, XP093014188, ISSN: 2688-4011, DOI: 10.1002/nano.202000055

## Description

### FIELD OF INVENTION

The subject matter of the present disclosure broadly relates to a layered composition and its preparation and particularly, refers to a bioactive layered hemostatic article for use in inhibiting excessive blood loss from a hemorrhaging site. Additionally, the present disclosure relates to a dressing material comprising the layered composition.

### BACKGROUND OF THE INVENTION

Haemorrhage, as a result of severe external injuries, occurs from an orifice or a deep wound in the body. The blood cells are incapable of forming clots in such traumatic wounds, leading to excessive blood loss from the body before receiving any sufficient clinical treatment. Extensive haemorrhage is one of the key factors for the increased mortality rate in traumatic injuries such as road accidents, bullet injuries on the battlefield, etc. In order to prevent such casualties, achieving rapid blood coagulation is of prime importance.

Several hemostats have been developed for the prevention of excessive haemorrhage. The first-line of local hemostats are oxidized cellulose and fibrin glue. Cellulose-based hemostats initiate blood clotting by contact activation of the coagulation cascade and providing a three-dimensional substrate for clot organization. Since these products do not contain any component of the intrinsic coagulation, they require a functional coagulation system, and hence, fail to achieve hemostasis in coagulopathic patients (J Biomed Mater Res A 82A (2) (2007) 274-280). Moreover, poor absorption of cellulose-based products causing postoperative healing complications, such as sepsis have been reported (Misch's Avoiding Complications in Oral Implantology, Elsevier Health Sciences (2017*);* The Lancet 380 (9847) (2012) 1099-1108*;* World Journal of Surgery 36 (2012) 2761-2766; World Journal of Surgery 31 (2007) 569-574).

Fibrin glue/sealants mimic the formation of fibrin clot by using a combination of fibrinogen and thrombin, wherein, fibrinogen and thrombin are dissolved in sterile water and dilute calcium chloride solution respectively in separate vials. Subsequently, the two solutions are injected simultaneously through a double-barreled syringe to form the clot. Although well accepted in surgical settings, these products are complicated to use and expensive, and hence not favorable to be used in emergency assistance or pre-hospital setting.

Local hemostats, such as zeolites and chitosan have also been investigated for controlling haemorrhage. Zeolites work by absorbing water from the bleeding site and promoting the concentration of coagulation factors and platelets, thereby leading to blood clotting. However, this water absorption process is highly exothermic that causes inflammation at the wound site (British Journal of Surgery 95(10) (2008) 1197-1225).

Chitosan triggers the protein cascade of the extrinsic coagulation pathway by rapidly activating and aggregating platelets attributed to its positively charged groups. Nonetheless, they are only capable of preventing low-pressure atrial bleeds. *(*The Journal of Trauma: Injury, Infection, and Critical Care 67(3) (2009) 450-460*).* Additionally, chitosan, because of its poly(cationic) nature, has been reported to possess mucoadhesive properties which may cause rebleeding during product removal (International Journal of Pharmaceutics 166(1) (1998) 75-88*;* Journal of Bioactive and Compatible Polymers 21(1) (2006) 39-54).

Z. Karahaliloǧlu *et al.,* reported a nano/micro bilayer hemostatic dressing comprising a porous bottom sublayer of crosslinked chitosan and bacterial cellulose doped with active agents such as calcium ions, vitamin K, kaolin, etc., and a nanofibrillar upper layer from silk fibroin (SF). It discloses a fabrication process involving several reagents and techniques, making it complex to be implemented on an industrial scale (Journal of Applied Polymer Science 133(28) (2016) 43567).

US 20110311632 A1 discloses a stable hemostatic product and composition prepared from crosslinked chitosan and a non-volatile plasticizer and a method for preparing the product by introducing dried chitosan into a package made of foil LDPE peelable laminate. The chitosan product effectively stopped bleeding during a 5 minute trial of venous bleeding in an aggressively anti-coagulated porcine model.

CN106178066A discloses a modified cellulose/chitosan compound hemostatic material comprising impregnating or coating modified cellulose with an acidic aqueous solution of chitosan. It also discloses modification of cellulose using one or more oxidant, esterifying reagent, etherifying reagent and concentrated sulphuric acid.

US 9 433 698 describes a composite laminate material which can be used to stop haemorrhage. The material comprises a chitosan based layer and a silk fibroin based layer. The ratio of carbohydrate to protein ranges from 1:1.5 to 1:2.5.

Although considerable types of hemostats and hemostatic composition are available these days, such as chitosan-based hemostatic composite with gelatin, collagen, etc., there are several limitations in terms of high manufacturing costs, inability to scale up, rebleeding during removal and inability to achieve rapid hemostasis, thereby failing in clinical translation.

Therefore, there is an emerging need for developing a facile and scalable process for the preparation of an inexpensive composition capable of achieving rapid hemostasis and increased clot firmness that could prevent rebleeding.

### SUMMARY OF THE INVENTION

In the first aspect of the present disclosure, there is provided a layered composition comprising: (a) chitosan as a base layer; and (b) a nanocomposite layer as a bioactive layer, wherein the nanocomposite layer comprises silica nanoparticles dispersed in silk fibroin matrix, wherein the base layer to the bioactive layer thickness is in a ratio range of 5:1 to 15:1, and wherein the weight ratio of silica nanoparticles: silk fibroin: chitosan is in a range of 0.1:1:1 to 10:20:20.

In the second aspect of the present disclosure, there is provided a process for preparing the layered composition of the first aspect comprising (1) chitosan as a base layer; and (2) a nanocomposite layer as a bioactive layer, the process comprising: (a) contacting chitosan in a solvent and freezing to obtain a base layer; (b) obtaining a first solution of silk fibroin in water; (c) dispersing silica nanoparticles in the first solution to obtain a second solution; (d) pouring the second solution on the base layer and freezing to obtain a bioactive layer on the base layer, and (e) freeze-drying the bioactive layer on the base layer obtained in the step (d) to obtain a layered composition.

In third aspect of the present disclosure, there is provided a hemostatic article comprising the layered composition of the first aspect, the layered composition comprising: (a) chitosan as a base layer; and (b) a nanocomposite layer as a bioactive layer, wherein the nanocomposite layer comprises silica nanoparticles dispersed in silk fibroin.

In fourth aspect of the present disclosure, there is provided the layered composition of the first aspect or the hemostatic article of the third aspect for use in a method for inhibiting loss of blood from a haemorrhaging site, said method comprising: (a) obtaining the layered composition or the hemostatic article; and (b) applying the composition or the hemostatic article to the haemorrhaging site for inhibiting loss of blood from the haemorrhaging site.

These and other features, aspects, and advantages of the present subject matter will be better understood with reference to the following description and appended claims.

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

### BRIEF DESCRIPTION OF THE DRAWINGS

Figure 1 depicts (a) scanning electron microscopic (SEM) image of silica nanoparticles and particle size distribution (inset); b) the powder X-ray diffraction pattern of silica nanoparticles; and c) the FTIR spectra of silica nanoparticles, in accordance with an implementation of the present disclosure.
Figure 2 depicts a) the blood-compatibility (hemocompatibility) assay of silica nanoparticles, and b) the corresponding percentage hemolysis, in accordance with an implementation of the present disclosure.
Figure 3 depicts the quantification of the cell viability assessment of silica nanoparticles by MTT assay, in accordance with an implementation of the present disclosure.
Figure 4 (a-e) depicts cell viability revealed by live(Calcein)/dead(EthD) assay stained in green/red for a) untreated control; b) cells treated with 50 µg/mL silica particles; c) cells treated with 100 µg/mL silica particles; d) cells treated with 200 µg/mL silica particles; e) cells treated with 500 µg/mL silica particles, in accordance with an implementation of the present disclosure.

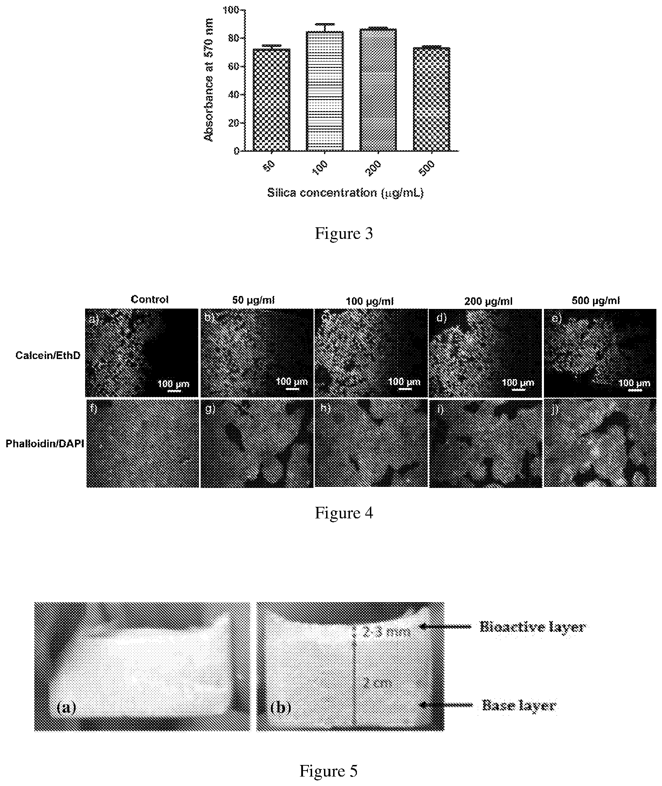

Figure 4 (f-j) depicts cell morphology revealed by Phalloidin(F-actin)/DAPI(nuclei) staining for f) untreated control; g) cells treated with 50 µg/mL silica particles; h) cells treated with 100 µg/mL silica particles; i) cells treated with 200 µg/mL silica particles; j) cells treated with 500 µg/mL silica particles, in accordance with an implementation of the present disclosure.
Figure 5 depicts photographic images of (a) pure chitosan; and (b) the layered hemostatic composition, in accordance with an implementation of the present disclosure.

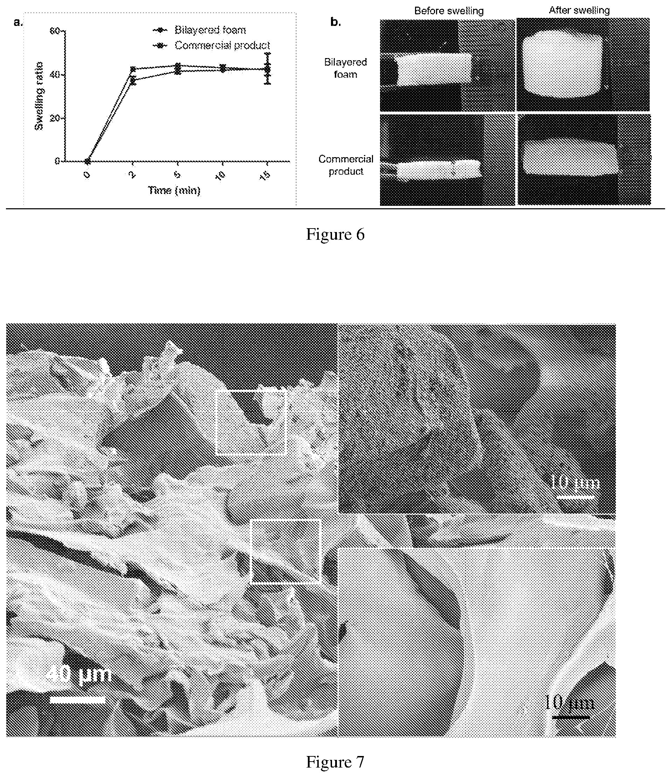

Figure 6 depicts a) a graphical representation of the swelling ratio of layered composition (bilayered foam) in comparison to the commercial product, b) photographic images of the two foams before and after saturation, in accordance with an implementation of the present disclosure.
Figure 7 depicts SEM micrograph of the bilayered foam (cross-sectional view), top layer shows silica particles-incorporated into the silk fibroin matrix (top inset) and bottom chitosan layer (bottom inset), in accordance with an implementation of the present disclosure.

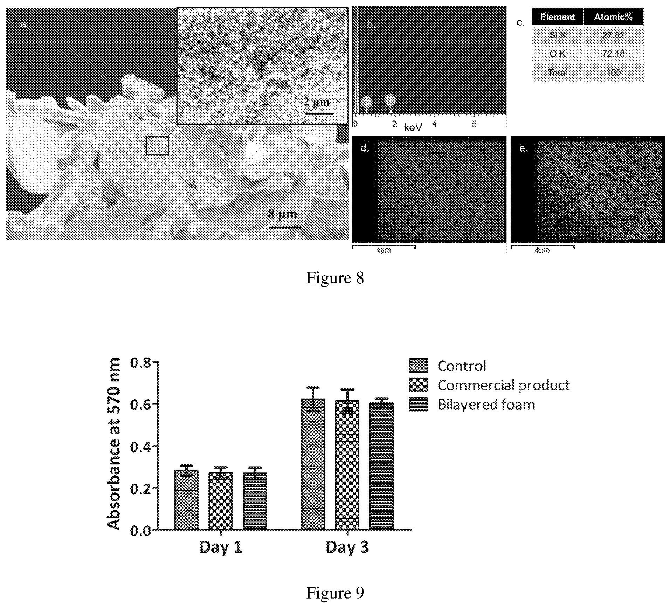

Figure 8 depicts the top layer of the bilayered foam (a) and the corresponding EDS (energy-dispersive x-ray spectrum) spectrum (b), atomic percentages (c) and elemental mapping of oxygen (d) and silicon (e), in accordance with an implementation of the present disclosure.
Figure 9 depicts quantification of cell viability by MTT assay on day 1 and day 3 after the treatment with conditioned media prepared with the bilayered foam and the commercial product, in accordance with an implementation of the present disclosure.

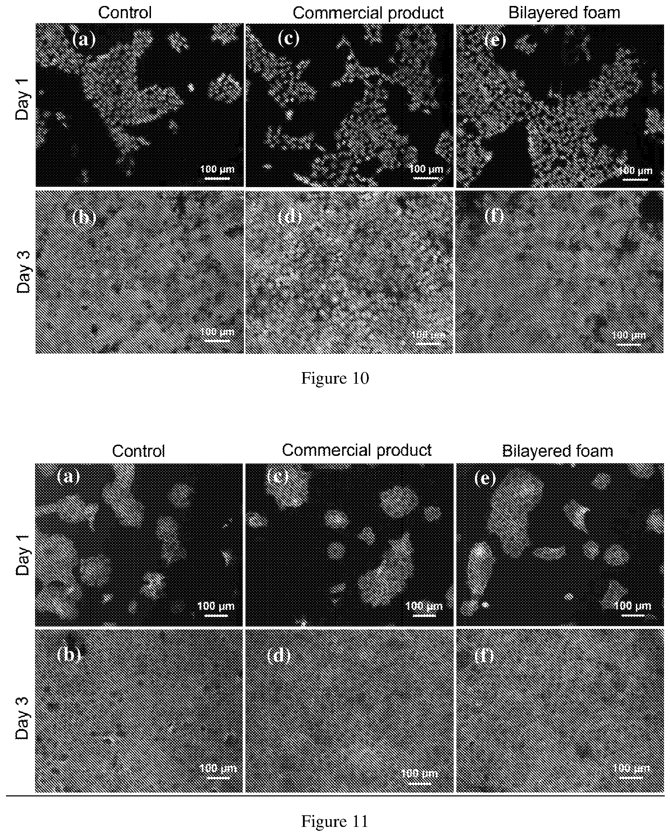

Figure 10 depicts the cell viability in the fluorescence images taken after Live(green)/Dead(red) staining of the cells on Day 1 and Day 3 for the commercial product (c, d) and the bilayered foam (e, f) in comparison with the untreated control group (a, b), in accordance with an implementation of the present disclosure.
Figure 11 depicts the cellular morphology by staining with Phalloidin (F-actin in green)/DAPI (nucleus in blue) on Day 1 and Day 3 for the commercial product (c, d) and the bilayered foam (e, f) in comparison with the untreated control group (a, b), in accordance with an implementation of the present disclosure.

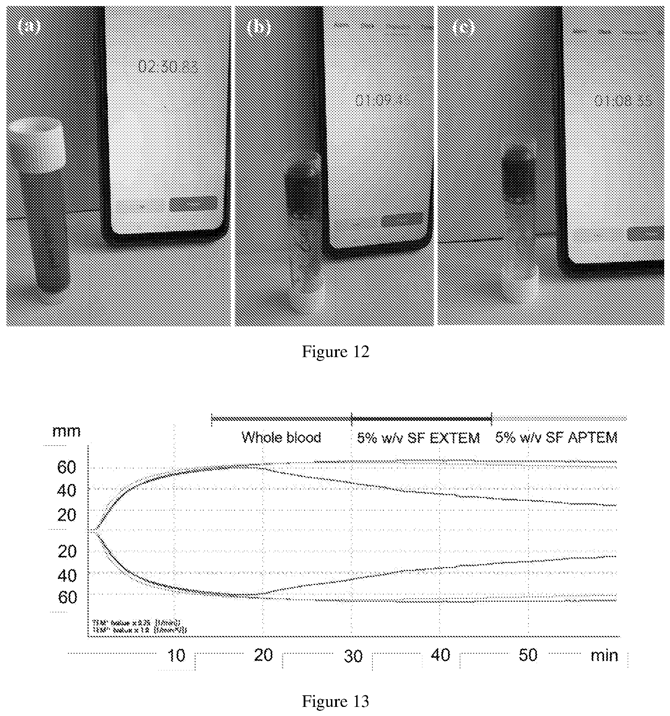

Figure 12 depicts the clotting time of (a) untreated whole rat blood, (b) commercial product, and (c) bilayered composition using tube inversion technique, in accordance with an implementation of the present disclosure.
Figure 13 depicts the thromboelastometric curves of 5% w/v SF in EXTEM (blue) and APTEM (green) in comparison with untreated whole blood (red), in accordance with an implementation of the present disclosure.

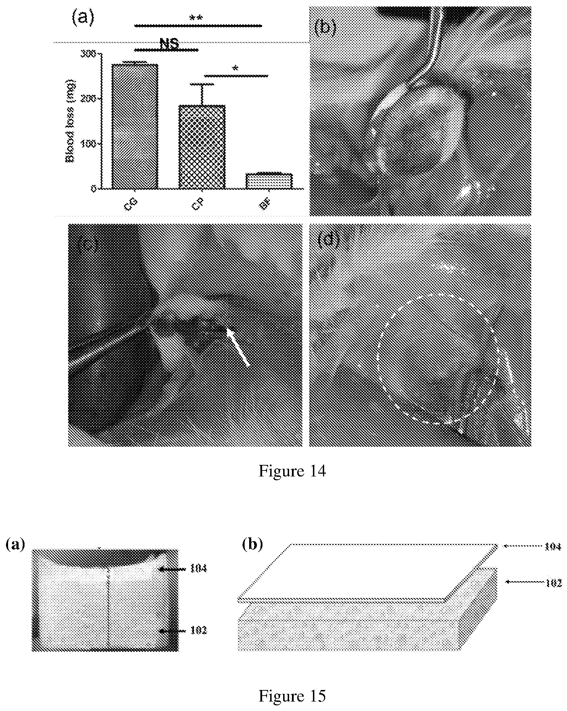

Figure 14 depicts the data obtained from the *in vivo* study, wherein, a femoral arterial bleed was created in a rodent model and treated with different products i.e. cotton gauze (CG), commercial product (CP) and bilayered foam (BF), (a) blood loss quantified at 120 s.,(*p<0.05, **p<0.001), (b) non-adhesive property of layered composition (bilayered foam), (c) muco-adhesive nature of commercial product that cannot be left behind, (d) remnants of the commercial product at the bleeding site post-application, in accordance with an implementation of the present disclosure.
Figure 15 depicts the (a) hemostatic article and (b) schematic representation of the hemostatic article comprising the base layer (102) and the bioactive layer (104), in accordance with an implementation of the present disclosure.

### DETAILED DESCRIPTION OF THE INVENTION

The disclosure includes all steps, features, compositions, and compounds referred to or indicated in this specification, individually or collectively, and any and all combinations of any or more of such steps or features.

### Definitions

For convenience, before further description of the present disclosure, certain terms employed in the specification, and examples are delineated here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

The term "layered composition" used herein refers to a composition comprising a base layer and a bioactive layer, and the composition is also referred to as bilayered composition. The base layer is a polysaccharide, in specific, chitosan; and the bioactive layer is a nanocomposite layer comprising silica nanoparticles dispersed in a biocompatible hydrophilic polymer (i.e.,) silk fibroin. The layered composition is also referred to as bilayered foam, hemostatic composition, hemostat composition, layered hemostatic composition, hemostat, or hemostatic article, and said terms are used interchangeably.

The term "nanoparticles" used herein refers to nanoscale material that are dispersed in a biocompatible hydrophilic polymer silk fibroin, capable of achieving hemostasis. In the present disclosure, the nanoparticle includes silica. For the purpose of the present disclosure, the layered composition comprises silica nanoparticles in the size of 10 to 1000 nm. The terms nanoparticles and particles are used interchangeably.

The term "biocompatible hydrophilic polymer" used herein refers to the polymeric material which are soluble in or absorb water and are non-toxic to biological tissues. In the present disclosure, the biocompatible hydrophilic polymer includes silk fibroin. For the purpose of the present disclosure, the layered composition comprises silk fibroin as the biocompatible hydrophilic polymer which has silica nanoparticles dispersed in it.

The term "freeze drying" used herein refers to the method of removal of solvent from materials under low temperature and pressure. The method is used to remove the solvent within the material and obtain a porous structure.

The term "cytocompatibility" used herein refers to the property of a substance to exhibit non-toxic bioactivity towards mammalian cells.

The term "cell viability" used herein refers to the fraction of number of living mammalian cells upon interaction with a substance. It is usually expressed in percentage.

The term "wound site" or "wound" or "haemorrhaging site" used herein refers to any site on a body of any animal or human that is wounded or has undergone a physical trauma or a surgical procedure leading to loss of blood. In the present disclosure, the wound includes, but not limited to, an arterial puncture wound, a venous puncture wound, arterial laceration wound, and a venous laceration wound.

The term "haemorrhaging" used herein refers to excessive loss of blood.

The term "chitosan derivatives" used herein refers to the compounds comprising one or more substituted groups on the structure of chitosan, obtained from chemical modification of the active functional groups on chitosan. In the present disclosure, the chitosan derivatives include, but not limited to, quaternary ammonium chitosan, carboxylated chitosan, acylated chitosan, and alkylated chitosan.

The term "commercial product" used herein refers a composition comprising chitosan. The commercial product refers to a chitosan foam.

Ratios, concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a temperature range of -196 °C to -4 °C should be interpreted to include not only the explicitly recited limits of -196 °C to about -4 °C, but also to include subranges, such as -75 °C to -15 °C, -60 °C to -8 °C and so forth, as well as individual amounts, including fractional amounts, within the specified ranges, such as -9.4 °C, and -25.8 °C, for example.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods, and materials are now described.

As discussed in the background, the current available hemostats face several limitations including lack of intrinsic coagulation, failure to achieve rapid hemostasis, complicated to use, expensive, rebleeding upon removal, postoperative healing complications, complex process of fabrication and inability to scale up. This further makes the hemostats and the process of preparation involved economically not feasible for use.

In view of the aforementioned shortcomings, it can be understood that an effective and inexpensive hemostatic composition is required which is easy and safe to use without causing any postoperative complications. The present disclosure provides a layered hemostatic composition, comprising chitosan (a polysaccharide) as a base layer, and a nanocomposite layer as a bioactive layer, which comprises bio-functional silica nanoparticles dispersed in silk fibroin as a biocompatible hydrophilic polymer. The nanocomposite layer is a top thin layer which is the first layer to come in contact with the wound site. The nanoparticles activate the intrinsic coagulation pathway to form a firm blood clot. The bottom thick chitosan layer acts as a base layer which essentially adds the absorption capacity to the layered composition while having pro-coagulant properties by activating the extrinsic coagulation pathway. Thus, the simultaneous activation of both the coagulation pathways by the layered hemostatic composition is achieved in the present disclosure, thereby, shortening the clotting time and preventing rebleeding. The present disclosure also provides an efficient, easy and scalable process required in order to obtain the layered hemostatic composition. This is achieved in the present disclosure using a facile two-step freezing and freeze-drying process.

The present disclosure is not to be limited in scope by the specific embodiments described herein, which are intended for the purposes of exemplification only.

In an embodiment of the present disclosure, there is provided a layered composition comprising: (a) chitosan as a base layer; and (b) a nanocomposite layer as a bioactive layer, wherein the nanocomposite layer comprises silica nanoparticles dispersed in silk fibroin.

The base layer to the bioactive layer thickness is in a ratio range of 5:1 to 15:1. In another embodiment of the present disclosure, the base layer to the bioactive layer thickness is in a ratio range of 6:1 to 13:1. In yet another embodiment of the present disclosure, the base layer to the bioactive layer thickness is in a ratio of 10:1.

In an embodiment of the present disclosure, there is provided a layered composition as disclosed herein, wherein the bioactive layer thickness is 2-3 mm and the base layer thickness is 2 cm.

The weight ratio of silica nanoparticles: silk fibroin: chitosan is in a range of 0.1:1:1 to 10:20:20. In another embodiment of the present disclosure, the weight ratio of silica nanoparticles: silk fibroin: chitosan is in a range of 0.5:1:1 to 5:5:15.

Thus according to the claimed invention, there is provided a layered composition comprising: (a) chitosan as a base layer; and (b) a nanocomposite layer as a bioactive layer, wherein the nanocomposite layer comprises silica nanoparticles dispersed in silk fibroin; and the base layer to the bioactive layer thickness is in a ratio range of 5:1 to 15:1; and weight ratio of silica nanoparticles: silk fibroin: chitosan is in a range of 0.1:1:1 to 10:20:20.

In an embodiment of the present disclosure, there is provided a layered composition comprising: (a) chitosan as a base layer in the range of 1 to 20 weight % with respect to the composition; and (b) a nanocomposite layer as a bioactive layer, wherein the nanocomposite layer comprises silica nanoparticles in a weight range of 0.1 to 10 weight % with respect to the composition, dispersed in silk fibroin in a range of 1 to 5 weight % with respect to the composition.

In an embodiment of the present disclosure, there is provided a layered composition as disclosed herein, wherein silica nanoparticles are in a size range of 10 to 1000 nm. In another embodiment of the present disclosure, the nanoparticles are in the size range of 45 to 775 nm. In yet another embodiment of the present disclosure, the nanoparticles are in the size range of 300 to 500 nm. The layered composition described herein is for use in inhibiting loss of blood from a haemorrhaging site.

In an embodiment of the present disclosure, there is provided a layered composition as disclosed herein, wherein the composition is in a form selected from foam, bandage, gauze, plaster, or lint. In another embodiment of the present disclosure, the composition is in a form selected from foam, bandage, or gauze. In yet another embodiment of the present disclosure, the composition is in a form selected from plaster, or lint.

In an embodiment of the present disclosure, there is provided a process for preparing the composition as disclosed herein, the process comprising: (a) contacting chitosan in a solvent and freezing to obtain a base layer; (b) obtaining a first solution of silk fibroin in water; (c) dispersing silica nanoparticles in the first solution to obtain a second solution; (d) pouring the second solution on the base layer and freezing to obtain a bioactive layer on the base layer, and (e) freeze-drying the bioactive layer on the base layer obtained in the step (d) to obtain a layered composition.

In an embodiment of the present disclosure, there is provided a process as disclosed herein, wherein silica nanoparticles are dispersed in water prior to dispersing in the first solution.

In an embodiment of the present disclosure, there is provided a process as disclosed herein, wherein the solvent is selected from acetic acid, water, or combinations thereof. In another embodiment of the present disclosure, the solvent is 1% acetic acid in water.

In an embodiment of the present disclosure, there is provided a process as disclosed herein, wherein chitosan is in a range of 1 to 20 % w/v with respect to the solvent, silk fibroin is in a range of 1 to 20 % w/v with respect to water; and silica nanoparticles is in a range of 0.1 to 10 % w/v with respect to water. In another embodiment of the present disclosure, wherein chitosan is in a range of 1 to 18 % w/v with respect to the solvent; silk fibroin is in a range of 1 to 15 % w/v with respect to water; and silica nanoparticles is in a range of 0.5 to 8 % w/v with respect to water.

In an embodiment of the present disclosure, there is provided a process for preparing the composition as disclosed herein, the process comprising: (a) contacting chitosan in a solvent and freezing to obtain a base layer; (b) obtaining a first solution of silk fibroin in water; (c) dispersing silica nanoparticles in the first solution to obtain a second solution; (d) pouring the second solution on the base layer and freezing to obtain a bioactive layer on the base layer, and (e) freeze-drying the bioactive layer on the base layer obtained in the step (d) to obtain a layered composition, wherein weight percentage ratio range of chitosan in the solvent is in a range of 1.5:1 to 3:1; and the first solution comprises 1 to 5 weight % of the silk fibroin in water.

In an embodiment of the present disclosure, there is provided a process for preparing the composition as disclosed herein, wherein freezing is carried out at a temperature in a range of -196 °C to -4 °C at atmospheric pressure for a time period in the range of 0.1 to 15 hours. In another embodiment of the present disclosure, freezing is carried out at a temperature in the range of -75 °C to -5 °C for a time period in the range of 2 to 13 hours.

In an embodiment of the present disclosure, there is provided a process for preparing the composition as disclosed herein, the process comprising: (a) contacting chitosan in a solvent and freezing at a temperature in the range of -196 °C to -4 °C for a time period in the range of 0.1 to 15 hours to obtain a base layer; (b) obtaining a first solution of silk fibroin in water; (c) dispersing silica nanoparticles in the first solution to obtain a second solution; (d) pouring the second solution on the base layer and freezing at a temperature in the range of -196 °C to -4 °C for a time period in the range of 0.1 to 15 hours to obtain a bioactive layer on the base layer, and (e) freeze-drying the bioactive layer on the base layer obtained in the step (d) to obtain a layered composition.

In an embodiment of the present disclosure, there is provided a process for preparing the composition as disclosed herein, wherein freeze-drying is carried out at a temperature in a range of -90 °C to 0 °C for a time period in the range of 12 to 40 hours at a pressure in a range of 0.1 to 1 mbar. In another embodiment of the present disclosure, freeze-drying is carried out at a temperature in a range of -85 °C to 0 °C for a time period in the range of 18 to 38 hours at a pressure in a range of 0.1 to 0.8 mbar.

In an embodiment of the present disclosure, there is provided a process for preparing the composition as disclosed herein, the process comprising: (a) contacting chitosan in a solvent selected from acetic acid, water, or combinations thereof, and freezing at a temperature in a range of -196 °C to -4 °C for a time period in a range of 0.1 to 15 hours to obtain a base layer; (b) obtaining a first solution of silk fibroin in water; (c) dispersing silica nanoparticles in the first solution to obtain a second solution; (d) pouring the second solution on the base layer and freezing at a temperature in a range of -196 °C to -4 °C for a time period in a range of 0.1 to 15 hours to obtain a bioactive layer on the base layer, and (e) freeze-drying the bioactive layer on the base layer obtained in the step (d) at a temperature in the range of -90 °C to 0 °C for a time period in a range of 12 to 40 hours at a pressure in a range of 0.1 to 1 mbar, to obtain a layered composition.

In an embodiment of the present disclosure, there is provided a process for preparing the composition as disclosed herein, wherein the layered composition is subjected to compression; and the compression is carried out in presence of a hydraulic or rolling press at a temperature in a range of 20 °C to 40 °C.

In an embodiment of the present disclosure, there is provided a process for preparing the composition as disclosed herein, wherein the layered composition is subjected to compression; and the compression is carried out in presence of a hydraulic or rolling press at a temperature in the range of 20 °C to 40 °C, and the layered composition is compressed by 1.5-10 fold. This step is employed to achieve definite pore size and density.

In an embodiment of the present disclosure, there is provided a process for preparing the composition as disclosed herein, the process comprising: (a) contacting chitosan in a solvent and freezing to obtain a base layer; (b) obtaining a first solution of silk fibroin in water; (c) dispersing silica nanoparticles in the first solution to obtain a second solution; (d) pouring the second solution on the base layer and freezing to obtain a bioactive layer on the base layer, and (e) freeze-drying the bioactive layer on the base layer obtained in the step (d) to obtain a layered composition, wherein the layered composition is subjected to compression; and the compression is carried out in presence of a hydraulic or rolling press at a temperature in the range of 20 °C to 40 °C, and the layered composition is compressed by 1.5-10 fold.

In an embodiment of the present disclosure, there is provided a hemostatic article comprising a layered composition as disclosed herein.

In an embodiment of the present disclosure, there is provided a hemostatic article as disclosed herein, wherein the hemostatic article is in a form selected from foam, bandage, gauze, plaster, or lint. In another embodiment of the present disclosure, the hemostatic article is in a form of foam. In one another embodiment of the present disclosure, the hemostatic article is in a form of a bandage. In yet another embodiment of the present disclosure, the hemostatic article is in a form of gauze. In another embodiment of the present disclosure, the hemostatic article is in a form of plaster. In one another embodiment of the present disclosure, the hemostatic article is in a form of lint.

In an embodiment of the present disclosure, there is provided a hemostatic article as described herein, which may further comprise an agent selected from the group consisting of analgesics, steroids, antihistamines, anesthetics, bactericides, disinfectants, fungicides, vasoconstrictors, hemostatics, chemotherapeutic drugs, antibiotics, keratolytics, cauterizing agents, antiviral drugs, epidermal growth factor, fibroblast growth factors, transforming growth factors, glycoproteins, fibrinogen, fibrin, humectants, preservatives, lymphokines, cytokines, odor controlling materials, vitamins, and clotting factors.

In an embodiment of the present disclosure, there is provided a hemostatic article comprising the layered composition as disclosed herein, and wherein the hemostatic article is in a form selected from foam, bandage, gauze, plaster, or lint.

In an embodiment of the present disclosure, there is provided the layered composition as disclosed herein for use in inhibiting loss of blood from a haemorrhaging site.

In an embodiment of the present disclosure, there is provided the hemostatic article as disclosed herein for use in inhibiting loss of blood from a haemorrhaging site.

Also disclosed herein is the hemostatic article comprising the composition as disclosed herein for use in inhibiting loss of blood from a haemorrhaging site, wherein the hemostatic article is in a form selected from foam, bandage, gauze, plaster, or lint.

Although the subject matter has been described in considerable detail with reference to certain examples and implementations thereof, other implementations are possible.

### EXAMPLES

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein. It is to be understood that this disclosure is not limited to particular methods, and experimental conditions described, as such methods and conditions may apply.

The forthcoming examples explain how the present disclosure provides a process for preparing a layered composition, the composition comprising a top thin nanocomposite layer made up of silica nanoparticles and silk fibroin as a bioactive layer; and a bottom thick polysaccharide layer of chitosan as a base layer.

Chitosan is a natural polysaccharide with pro-coagulant and absorption capability, which is available commercially as a hemostat for low atrial bleeding. Nonetheless, due to the polycationic nature of chitosan, it possesses mucoadhesive property, which causes rebleeding while removal. In addition, chitosan does not dissolve in neutral aqueous solution and requires acidic solvent, which leads to cytotoxicity and irritation when in contact at the wound site. Silica nanoparticles have a negatively-charged surface and are a potent activator of the intrinsic coagulation cascade. However, silica is also well known to possess adhesive ability with biological tissues, which can cause rebleeding upon its removal.

In order to address the abovementioned drawbacks of adhesiveness and rebleeding caused by silica and chitosan at the wound site, the present disclosure provides dispersion of silica nanoparticles in silk fibroin, forming a bioactive layer. Silk fibroin is a naturally abundant protein, where the amino acid sequence of silk fibroin contains repetition of "Gly-Ala-Gly-Ala-Gly-Ser" which can self-assemble into the β-sheet structure. Silk fibroin possesses remarkable mechanical properties, biocompatibility, blood compatibility, controllable degradation, water absorption, and gel-forming capability.

The bioactive layer (silica nanoparticles and silk fibroin) comprising silk fibroin is a biocompatible hydrophilic polymer, which is well known for exhibiting wound healing activity. This layer provides the added benefit of avoiding direct contact of acidic solvent used for chitosan dissolution with the wound site.

### Materials and Methods

For the purpose of the present disclosure, following raw materials with the specified grades/brands were used.
a) Chitosan - Sigma-Aldrich (300-350 kDa);
b) Silk Fibroin - Extracted from silkworm cocoons;
c) PBS - Thermo Fisher Scientific; and
d) Triton X-100 - Sigma-Aldrich;

The synthesized silica nanoparticles were characterized by FTIR spectroscopy (Perkin-Elmer, FTIR-ATR). The crystallinity of the synthesized silica nanoparticles was characterized by powder X-ray diffraction (X-Pert PRO, PANalytical). The morphology was examined by scanning electron microscopy (Ultra55 FE-SEM Karl Zeiss EDS). The blood clotting time of individual components was determined using rotational thermoelectrometry (ROTEM) (Sonoclot^{®} analyzer).

### EXAMPLE 1

### Preparation and Characterization of Silica (SiO₂) Nanoparticles

The silica nanoparticles were prepared using sol-gel method (J. Am. Sci 6(11) (2010) 985-989). The method involves hydrolysing silica precursor (tetraethyl orthosilicate, TEOS) in the presence of acid or base, which forms a dispersed solution, followed by the gelation step. In an example, silica nanoparticles were subjected to physicochemical and biological characterization.

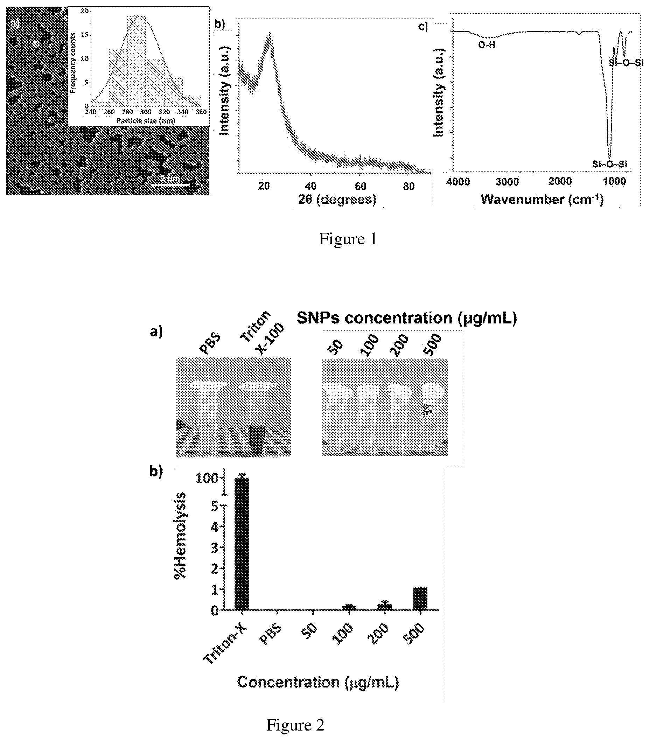

Figure 1a depicts the SEM images of the silica nanoparticles. The formation of silica nanoparticles was confirmed by the two major peaks of the FTIR spectrum, as shown in Figure 1(c). The peak observed at 1077 cm⁻¹ corresponds to the asymmetric stretching of O-Si-O, whereas the peak 798 cm⁻¹ indicates the symmetric vibration of O-Si-O (Azarshin, S., et al. Surface functionalization of silica nanoparticles to improve the performance of water flooding in oil wet reservoirs. Energy Explor. Exploit. 35, 685-697 (2017).

The crystallinity of the as-synthesized silica nanoparticles was characterized by powder X-ray diffraction (XRD) with a 2θ angle from 10 to 90, as shown in Figure 1(b). The XRD pattern indicates the amorphous nature of silica nanoparticles (Gholami, T., et al, Synthesis and characterization of spherical silica nanoparticles by modified Stöber process assisted by organic ligand. Superlattices Microstruct. 61, 33-41 (2013). The broad peak observed at θ = 23° represents the characteristic silica peak.

Particle size of the silica nanoparticles was measured by dynamic light scattering technique (DLS) (Figure la(inset)). Table 1 shows that the average particle size of silica nanoparticles was 335 ± 13 nm with polydispersity index (PDI) value of 0.221 ± 0.07 which indicated the monodispersity of the nanoparticles. Measurement of the zeta potential values provided a measurable information about the charge present on the surface of the nanoparticles. The use of silica nanoparticles is intended to initiate the intrinsic coagulation cascade through its highly negative surface charge, which can be achieved by the prepared silica nanoparticles as it exhibits the zeta potential value of -45.9 ± 1.1 mV. (Jiang, L. et al. Silica nanoparticles induced the pre-thrombotic state in rats via activation of coagulation factor XII and the JNK-NF-κB/AP-1 pathway. Toxicol. Res. (Camb). 4, 1453-1464 (2015).)

**Table 1**

| | **Particle size (nm) (Mean ± SD)** | **PDI (Mean ± SD)** | **Zeta Potential (mV) (Mean ± SD)** |
|---|---|---|---|
| SiO₂ nanoparticles | 335.1 ± 13.1 | 0.221 ± 0.07 | -45.9 ± 1.1 |

The morphology was examined by SEM. The spherical shape of the silica nanoparticles was evident from the SEM images as shown in Figure 1(a). It is observed that the particles are nearly monodisperse in agreement with the polydispersity index.

To assess the blood compatibility of the silica nanoparticles a blood compatibility assay was performed using PBS as a positive control, Triton X-100 as a negative control and different concentration of the silica nanoparticles at 50, 100, 200 and 500 µg/ml, as samples. The hemolysis data of the silica nanoparticles is shown in Figures 2a and 2b. Triton X-100, which is a well-known cell lysis agent, ruptured the blood cells as the supernatant is intensively red in color, whereas PBS (pH 7.4) did not show any hemolysis. The highest concentration of silica nanoparticles (500 µg/ml) showed approximately 1.0% of hemolysis. A material is considered hemolytic if the value of %hemolysis is greater than 5%. (Dobrovolskaia, M. A. et al. Method for analysis of nanoparticle hemolytic properties in vitro. Nano Lett. 8, 2180-2187 (2008)). The concentration of silica nanoparticles used for the layered hemostatic composition is much lower than 500 µg/ml, and thus, it is expected to not have hemolytic effects.

Figure 3 depicts the quantification of cell viability by MTT assay after treatment with varying silica nanoparticles concentration (50 - 500 µg/mL). Acceptable cell viability was obtained upon treatment with silica nanoparticles even up to 500 µg/mL.

The cytocompatibility of silica nanoparticles was assessed against human keratinocytes (HaCaT) cells using Live/Dead (Calcein/EthD) staining. Different concentrations of silica nanoparticles at 50, 100, 200, and 500 µg/ml were taken as samples. The cell viability assessment of the nanoparticles at different concentrations is shown in Figure 4. It is observed from the percent cell viability data that the silica nanoparticles exhibit cytocompatibility even at high concentration of 500 µg/ml.

The cells treated with silica nanoparticles showed green fluorescence whereas no red fluorescence could be observed, which is very much similar to the control group with no silica exposure confirming the cytocompatibility of the silica nanoparticles up to 500 µg/ml, as shown in Figure 4(a-e). Phalloidin (green fluorescence)/ nuclear (blue fluorescence) staining was performed to check the effect of silica nanoparticles on the cellular morphology as shown in Figure 4(f-j). Normal, healthy cell morphology is observed in cells treated with silica nanoparticles up to 500 µg/ml and the control. Thus, silica nanoparticles are suitable to be used in a composition for providing procoagulant property. However, silica nanoparticles cannot be used alone as they have mucoadhesive property and are in particulate form making it difficult to use at the haemorrhaging site.

### EXAMPLE 2

### Preparation and Characterization of Layered Hemostatic Composition and Hemostatic Article CS/SF/SiO₂

In an example, the layered hemostatic composition was prepared from chitosan (CS) dissolved in a solvent and silica nanoparticles dispersed silk fibroin (SF/SiO₂) solution using a two-step freezing and freeze-drying layer-by-layer fabrication method. The prepared composition was characterized for cytocompatibility and blood clotting efficacy.

### Preparation of Layered Hemostatic Composition CS/SF/SiO₂

The solvent used for the process of the present disclosure is 1% v/v acetic acid in water. 2% w/v of chitosan (CS) was contacted with 1% v/v acetic acid was poured into a Teflon mold and frozen at a temperature lower than -20 °C for 12 hours to obtain a base layer. Silk fibroin (SF) was dissolved in water to form a first solution of 2% w/v of silk fibroin (SF). Silica nanoparticles were dispersed in 2% w/v of silk fibroin (SF) solution to obtain a second solution. The second solution was then poured onto the base layer such that the amount of silica nanoparticles in the composition is 1 mg per cm² of the layered composition. Subsequently, it was frozen at a temperature lower than -20 °C for 12 hours to obtain a bioactive layer on the base layer. Further, the bioactive layer on the base layer was freeze-dried at -80 °C and 0.2 mbar for 36 hours to obtain the layered hemostatic composition. The layered composition was then compressed by 1.5 to 10 fold using a hydraulic or rolling press, in the temperature range of 20 °C to 40 °C. The compression was done to achieve definite pore size and density of the layered hemostatic composition.

The prepared layered hemostatic composition CS/SF/SiO₂ comprises two layers including a fine layer of silica nanoparticles dispersed in silk fibroin (bioactive layer) and a thick layer of chitosan (base layer) as shown in Figure 5b. Figure 5a depicts only the base layer comprising chitosan. The layered composition comprises the bioactive layer of thickness 2-3 mm and the chitosan base layer underneath of 2 cm thickness as shown in Figure 5b. The swelling ratio of the layered composition was compared with the commercial hemostat product. The commercial product is a hemostatic foam comprising chitosan. Figure 6 depicts (a) graphical representation of the swelling ratio of layered composition (bilayered foam) in comparison to the commercial product, and (b) photographic images of the layered composition (bilayered foam) and the commercial product before and after saturation. Both the hemostatic foams i.e., the layered composition and the commercial product attained the maximum absorption capacity in the first 2 min. The readings were taken until saturation, i.e., at 15 mins. At saturation, the swelling ratio of the bilayered foam (45 ± 4.7) was not statistically different (p < 0.05) from that of the commercially available product (42 ± 2.6).

The dispersion of silica nanoparticles in silk fibroin provides the easy peelable effect which in turn reduces the toxicity and rebleeding issues. The presence of silk fibroin provides uniform dispersion of the silica nanoparticles and is available to the wound site with less cytotoxicity. It further prevents the direct contact of chitosan with the silica nanoparticles as chitosan requires the presence of acidic solvents like acetic acid for its dissolution.

### Characterization of Layered Hemostatic Composition CS/SF/SiO₂

SEM imaging of the layered hemostatic composition CS/SF/SiO₂ is shown in Figure 7. It is observed from the SEM images that there are two different layers of chitosan and silk fibroin, and both are highly porous in nature which is suitable to absorb a large amount of bodily fluid during its application. SEM micrograph of the bilayered foam (cross-sectional view, Figure 7), inset (top right of Figure 7) depicts top active layer showed silica nanoparticles in the silk fibroin matrix and inset (bottom right of Figure 7) depicts bottom layer of the layered composition (foam), a smooth chitosan layer. Thus, the SEM images confirmed the formation of bilayered hemostatic composition (CS/SF/SiO₂). Further the energy dispersive spectroscopy (EDS) spectrum of the layered composition of the SEM image (Figure 8a) depict the presence of Si (Figure 8d) and O (Figure 8e) and their atomic % as shown in Figure 8(b-c).

### In Vitro Cytocompatibility Studies

The cytocompatibility of the prepared hemostatic composition (bilayered foam) (CS/SF/SiO₂) and commercial product (hemostatic composition with CS) were assessed against keratinocytes (HaCaT). The cells were treated with conditioned media prepared by soaking the articles for 24 hours in a cell culture media (DMEM low glucose) at 5 mg/mL. The percent cell viability data on day 1 and day 3 as shown in Figure 9 indicates the cytocompatibility of the degraded products from the hemostatic composition. The data were subjected to statistical analysis using one-way ANOVA followed by Tukey's post-hoc test (p < 0.05) and no significant difference was observed between the control and the conditioned media treated groups comprising the hemostatic compositions. Hence, it was found that the layered hemostatic composition of the present disclosure was non-toxic to the cells.

The cytocompatibility of the layered composition was confirmed using Live/Dead staining. The cells in all the three groups, i.e., control, commercial product (hemostatic composition with CS) and the hemostatic layered composition (CS/SF/SiO₂), were stained with live/dead stains as shown in Figure 10(a-f), where calcein-AM and ethidium homodimer stain live and dead cells, respectively. Phalloidin (green fluorescence)/ nuclear (blue fluorescence) staining as shown in Figure 11(a-f) was performed to examine the effect of degraded products on the cellular morphology. Normal, healthy cell morphology could be observed in cells treated with the conditioned media comprising the compositions, and the control, further confirming the cytocompatibility of the hemostatic layered composition of the present disclosure.

### Blood Clotting Efficacy

The blood clotting efficacy of the layered hemostatic composition was compared to that of pure chitosan (CS) foam and the efficacy was examined using rat blood collected through retroorbital bleeding as shown in Figure 12 (a-c). The tube inversion technique was used wherein, 1 ml of blood was poured onto the equal weight of the hemostatic composition and CS foam and then, the tubes were inverted repeatedly until clot formation and the clotting time was noted. The untreated whole blood remained unclotted at 2.5 minutes whereas, both, the bilayered hemostatic composition and the commercial CS foam, coagulated around 1 minute, hence exhibiting the procoagulant activity.

### EXAMPLE 3

### Effect of Individual Components on Whole Blood Clotting Time

The example involves the study of the blood clotting time of all the individual components, i.e., silica nanoparticles, chitosan and silk fibroin. The time was determined using rotational thermoelectrometry (ROTEM).

The effect of the individual components on the extrinsic coagulation pathway was examined using EXTEM (extrinsic thermoelectrometry or extrinsic thromboelastometry), and APTEM (aprotinin thermoelectrometry) which mildly activates hemostasis. The untreated whole blood clotted in 59 seconds. Figure 13 depicts the thromboelastometric curves of 5% w/v SF in EXTEM (blue) and APTEM (green) in comparison with whole blood (red). Table 2 shows the clotting time of each component of the layered hemostatic composition.

**Table 2**

| **S. No.** | **Samples** | | **Blood clotting time (s)** |
|---|---|---|---|
| 1 | Whole blood | | 59 |
| 2 | SiO₂ nanoparticles | 2 mg/ml | 45 |
| | | 6 mg/ml | 40 |
| 3 | 1% Chitosan (CS) | | 45 |
| 4 | 1% Silk fibroin (SF) | | 58 |
| 5 | 2% Silk fibroin (SF) | | 55 |
| 6 | 5% Silk fibroin (SF) | | 58 |
| 7 | 5% Silk fibroin (SF) (APTEM) | | 52 |

It is observed that with an increase in the concentration of silica nanoparticles, the clotting time decreases from 59 seconds. The concentration could not be increased beyond 6 mg/mL as it would clot the blood within 10 seconds, i.e., before the ROTEM test could be performed. For chitosan, the clotting time reduced to 45 seconds. Overall, the ROTEM data confirms that silica nanoparticles and chitosan in the layered hemostatic composition possess pro-coagulant abilities. Silk fibroin does not alter the clotting behaviour of the whole blood, as shown in the ROTEM data (Table 2). Nonetheless, this property was shown to be concentration-dependent. The untreated whole blood clotted in 59 s and the addition of 2% w/v SF does not alter the clotting time (55 s). But an increase in SF concentration to 5% w/v led to an enhanced fibrinolytic effect (activation of plasmin). This phenomenon can be seen as an early dip in the thromboelastometric curve after maximum clot firmness (Figure 13). To confirm this fibrinolytic activity, aprotinin was added (APTEM) to inhibit the plasmin activity. The curve obtained in APTEM appeared similar to that of untreated blood. Hence, the results proved that a high concentration of SF (5% w/v) increases the possibility of early fibrinolysis. Therefore, the concentration of SF was fixed at 2% w/v in the bilayered composition.

Based on these data, the layered hemostatic composition was prepared using the concentration of SPs (silica nanoparticles) fixed at 1 mg/cm², accounting for maximum effective concentration of 150 µg/mL in the conditioned media, 2% w/v of chitosan (CS) and 2% w/v of silk fibroin (SF). 1 to 5 mg/cm² of silica was preferred since greater than 5 mg/ml silica possessed cytotoxic behaviour.

The lower concentration of chitosan allowed the flowability of the solution while pouring into the container, thereby, obtaining a solution that can be easily molded or poured into a container making it easier to scale up at an industrial scale. The intactness of the layered hemostatic composition is obtained at a certain concentration such that the composition can withstand the pressure applied while using the product on a bleeding site. On the other hand, if the concentration of chitosan is higher than 2% w/v, the solution tends to become more viscous, i.e., less flowable and the resulting layered composition becomes stiffer. Also, 2% w/v of silk fibroin was used because at higher concentration (5% w/v) fibrinolysis by plasmin was enhanced as verified by ROTEM. At a concentration lower than 2% w/v, silk fibroin formed a brittle layer and was not capable to hold the silica nanoparticles.

Further, though chitosan possesses procoagulant property, it is also mucoadhesive which could lead to rebleeding. Due to the adhesiveness, a part of the chitosan may be left behind at the bleeding site which could be a potential irritant inhibiting the healing of the wound site. Hence chitosan itself is not a suitable hemostat. Further, although silk fibroin is non-adhesive, cannot be used as hemostat since it does not carry clotting ability. Silica with silk fibroin itself does not form a thick foam structure, and further silk fibroin requires additional chemical/physical modification to fabricate a sturdy porous structure which complicates the fabrication process. A composition of silica with chitosan is also disadvantageous since both are mucoadhesive and higher are the chances of rebleeding and leaving behind remnants at the bleeding site preventing wound healing.

Therefore, a layered composition comprising silica in silk fibroin with chitosan as base layer as illustrated in the present disclosure inhibited blood loss at the haemorrhaging site as well as exhibited wound healing property. The bioactive layer wherein silica is dispersed in silk fibroin forms a non-adhesive layer and maintained hemostatic efficacy. Since this is a thin layer/coating, it does not require additional chemical/physical modification. Further, the base layer chitosan added to the absorption capacity and carried clotting ability. Thus, the layered composition of the present disclosure is a multifunctional hemostat composition which is cytocompatible and is also wound healing.

Further the clotting time achieved by the layered composition of the present disclosure is comparable to the commercial product as can be seen from Table 3 below. Clotting time of the layered composition and commercial product in comparison with untreated whole blood obtained by tube inversion technique is shown in Table *3. In vivo* study of clotting efficacy of layered composition and commercial product in comparison with untreated whole blood in femoral arterial bleed was studied and summarized in Table 4 below. Hemostasis could not be achieved using cotton gauze, whereas the commercial product and the bilayered foam could attain rapid clotting (30 s) (Table 4). Nonetheless, the commercial product caused rebleeding 50% of the time due to the adhesive property (Figure 14c). It was observed that, once the rebleeding occurred, the same test sample could not stop the profuse bleeding. Moreover, at the time of removal, some part of CP was left behind at the wound site (Figure 14d). Contrarily, removing the bilayered hemostatic foam (the layered composition) was easy, and no rebleeding was observed (Figure 14b). Additionally, there was a clear demarcation between the product and the underlying tissue; hence no product residue was left behind (Figure 14b).

**Table 3**

| Sample | Clotting time (s) |
|---|---|
| | (Mean ± SD) |
| Untreated whole blood | 250 ± 35 |
| Commercial product | 62 ± 6 |
| Layered composition | 60 ± 3 |

**Table 4:**

| Sample | Hemostasis | Clotting time (s) | %occurrence of rebleeding |
|---|---|---|---|
| Untreated whole blood | No | > 120 | NA |
| Cotton gauze (CG) | No | > 120 | NA |
| Commercial product (CP) | Yes | 30 | 50% |
| Bilayered foam (BF) | Yes | 30 | 0% |

Furthermore, the extent of blood loss measured at the end of 120 s is summarized in Figure 14a. Cotton gauze soaked the maximum amount of blood, followed by the commercial product. The bilayered foam absorbed the minimum amount of blood before achieving hemostasis and was significantly lower than the commercial product (p < 0.05) and cotton gauze (p < 0.001). This is because pure chitosan (commercial product) achieves hemostasis by moisture absorption, concentration of clotting factors, and aggregation of platelets. (Thatte, Hemant S. et al . Mechanisms of poly-N-acetyl glucosamine polymer-mediated hemostasis: platelet interactions. J. Trauma 57, p S13-S21, (2004).) On the other hand, in the bilayered foam, the active layer containing silica nanoparticles is the first to come in contact with the bleeding site. Silica nanoparticles trigger blood clotting by activating the intrinsic pathway (Jiang, L. et al. Silica nanoparticles induced the pre-thrombotic state in rats via activation of coagulation factor XII and the JNK-NF-κB/AP-1 pathway. Toxicol. Res. (Camb). 4, 1453-1464 (2015)), thereby achieving rapid clotting with minimal blood loss. Therefore, these results confirmed that silk fibroin creates a non-adhesive barrier while maintaining the clotting efficiency of silica nanoparticles and chitosan. It can be seen that the layered composition is better coagulant in comparison with untreated whole blood and cotton gauze. Though the clotting time of the commercial product and layered composition of the present disclosure is comparable, the other properties such as non-adhesiveness, ease of removal, and wound healing property makes the layered composition advantageous over the commercial product.

### EXAMPLE 4

### Hemostatic Article

A hemostatic article of the present disclosure comprises the layered hemostatic composition as prepared herein. Figure 15(a-b) shows the hemostatic article comprising a base layer (102) and a bioactive layer (104), along with an enlarged schematic representation of different layers of the hemostatic article. The hemostatic article of the present disclosure may further comprise an agent selected from the group consisting of analgesics, steroids, antihistamines, anesthetics, bactericides, disinfectants, fungicides, vasoconstrictors, hemostatics, chemotherapeutic drugs, antibiotics, keratolytics, cauterizing agents, antiviral drugs, epidermal growth factor, fibroblast growth factors, transforming growth factors, glycoproteins, fibrinogen, fibrin, humectants, preservatives, lymphokines, cytokines, odor controlling materials, vitamins, and clotting factors. These agents may be present in the bioactive layer 104. When the hemostatic article is applied to the wound site/ haemorrhaging site, it is the bioactive layer 104 which is in contact with the wound site/ haemorrhaging site.

### ADVANTAGES OF THE PRESENT DISCLOSURE

The present disclosure discloses a layered composition comprising a chitosan as a base layer and a nanocomposite layer as a bioactive layer, wherein the nanocomposite layer comprises silica nanoparticles dispersed in a silk fibroin, and a process for the preparation of said composition. The present disclosure also discloses a hemostatic article and a dressing material comprising said composition. The process for preparing the layered composition involves a layer-by-layer fabrication method which is a facile technique and can be easily scaled-up. This allows the application of the layered composition to be economically feasible and efficient in treatment of external haemorrhage.

The process of the present disclosure provides a low-cost layered hemostatic composition by selection of cheaper biocompatible materials yet resulting in lesser blood clotting time with advantages of increasing the clot firmness, safety and facile use without any postoperative complications. The present disclosure also provides a layered hemostatic article which overcomes the drawbacks associated with individual components such as high adhesiveness, rebleeding upon removal and prevents the contact of acidic solvent used for chitosan dissolution from the wound site, as the acidic solvent causes cytotoxicity and irritation at the wound bed.

## Claims

1. A layered composition comprising:
a) chitosan as a base layer; and
b) a nanocomposite layer as a bioactive layer,
wherein the nanocomposite layer comprises silica nanoparticles dispersed in silk fibroin,
wherein the base layer to the bioactive layer thickness is in a ratio range of 5:1 to 15:1, and
wherein weight ratio of silica nanoparticles: silk fibroin: chitosan is in a range of 0.1:1:1 to 10:20:20.

2. The composition as claimed in claim 1, wherein the nanoparticles are in the size range of 10 to 1000 nm.

3. The composition as claimed in claim 1, wherein the composition is a hemostatic composition.

4. The composition as claimed in any one of the claims 1 to 3, wherein the composition is in a form selected from foam, bandage, gauze, plaster, or lint.

5. A process for preparing the composition as claimed in claim 1, the process comprising:
a) contacting chitosan in a solvent and freezing to obtain a base layer;
b) obtaining a first solution of silk fibroin in water;
c) dispersing silica nanoparticles in the first solution to obtain a second solution;
d) pouring the second solution on the base layer and freezing to obtain a bioactive layer on the base layer, and
e) freeze-drying the bioactive layer on the base layer obtained in the step (d) to obtain a layered composition.

6. The process as claimed in claim 5, wherein the solvent is selected from acetic acid, water, or combinations thereof and silica nanoparticles are dispersed in water prior to dispersing in the first solution.

7. The process as claimed in claim 5, wherein freezing is carried out at a temperature in a range of -196 °C to -4 °C at atmospheric pressure for a time period in a range of 0.1 to 15 hours; and freeze-drying is carried out at a temperature in a range of -90 °C to 0 °C for a time period in a range of 12 to 40 hours at a pressure in a range of 0.1 to 1 mbar.

8. The process as claimed in claim 5, wherein the layered composition is subjected to compression; and the compression is carried out in presence of a hydraulic or rolling press at a temperature in the range of 20 °C to 40 °C.

9. A hemostatic article comprising the composition as claimed in claim 1.

10. The hemostatic article as claimed in claim 9, wherein the hemostatic article is in a form selected from foam, bandage, gauze, plaster, or lint.

11. The composition as claimed in claim 1 for use in inhibiting loss of blood from a haemorrhaging site.

12. The hemostatic article as claimed in claim 9, for use in inhibiting loss of blood from a haemorrhaging site.

## Patentansprüche

1. Eine geschichtete Zusammensetzung, umfassend:
a) Chitosan als Basisschicht; und
b) eine Nanokompositschicht als bioaktive Schicht,
wobei die Nanokompositschicht in Seidenfibroin dispergierte Siliciumdioxid-Nanopartikel umfasst,
wobei die Dicke der Basisschicht zu der Dicke der bioaktiven Schicht in einem Verhältnisbereich von 5: 1 bis 15:1 liegt, und
wobei das Gewichtsverhältnis von Siliciumdioxid-Nanopartikeln: Seidenfibroin: Chitosan in einem Bereich von 0.1:1:1 bis 10:20:20 liegt.

2. Die beanspruchte Zusammensetzung nach Anspruch 1, wobei die Nanopartikel in dem Größenbereich von 10 bis 1000 nm liegen.

3. Die beanspruchte Zusammensetzung nach Anspruch 1, wobei die Zusammensetzung eine hämostatische Zusammensetzung ist.

4. Die beanspruchte Zusammensetzung nach einem der Ansprüche 1 bis 3, wobei die Zusammensetzung in einer aus Schaumstoff, Verband, Gaze, Pflaster oder Scharpie ausgewählten Form vorliegt.

5. Ein Verfahren zur Herstellung der beanspruchten Zusammensetzung nach Anspruch 1, wobei das Verfahren Folgendes umfasst:
a) Inkontaktbringen von Chitosan mit einem Lösungsmittel und Einfrieren, um eine Basisschicht zu erhalten;
b) Erhalten einer ersten Lösung von Seidenfibroin in Wasser;
c) Dispergieren von Siliciumdioxid-Nanopartikeln in der ersten Lösung, um eine zweite Lösung zu erhalten;
d) Gießen der zweiten Lösung auf die Basisschicht und Einfrieren, um eine bioaktive Schicht auf der Basisschicht zu erhalten, und
e) Gefriertrocknen der in Schritt (d) erhaltenen bioaktiven Schicht auf der Basisschicht, um eine geschichtete Zusammensetzung zu erhalten.

6. Das beanspruchte Verfahren nach Anspruch 5, wobei das Lösungsmittel aus Essigsäure, Wasser oder Kombinationen davon ausgewählt ist und Siliciumdioxid-Nanopartikel vor dem Dispergieren in der ersten Lösung in Wasser dispergiert werden.

7. Das beanspruchte Verfahren nach Anspruch 5, wobei das Einfrieren bei einer Temperatur in einem Bereich von -196 °C bis -4 °C bei atmosphärischem Druck über eine Zeitdauer in einem Bereich von 0.1 bis 15 Stunden durchgeführt wird; und das Gefriertrocknen bei einer Temperatur in einem Bereich von -90 °C bis 0 °C über eine Zeitdauer in einem Bereich von 12 bis 40 Stunden bei einem Druck in einem Bereich von 0.1 bis 1 mbar durchgeführt wird.

8. Das beanspruchte Verfahren nach Anspruch 5, wobei die geschichtete Zusammensetzung einer Kompression unterzogen wird; und die Kompression in Gegenwart einer hydraulischen Presse oder Walzenpresse bei einer Temperatur im Bereich von 20 °C bis 40 °C durchgeführt wird.

9. Ein hämostatischer Artikel, umfassend die beanspruchte Zusammensetzung nach Anspruch 1.

10. Der hämostatische Artikel nach Anspruch 9, wobei der hämostatische Artikel in einer aus Schaumstoff, Verband, Gaze, Pflaster oder Scharpie ausgewählten Form vorliegt.

11. Die beanspruchte Zusammensetzung nach Anspruch 1 zur Verwendung bei der Hemmung des Verlusts von Blut aus einer Blutungsstelle.

12. Der hämostatische Artikel nach Anspruch 9 zur Verwendung bei der Hemmung des Verlusts von Blut aus einer Blutungsstelle.

## Revendications

1. Une composition stratifiée comprenant :
a) du chitosane en tant que couche de base ; et
b) une couche nanocomposite en tant que couche bioactive,
dans laquelle la couche nanocomposite comprend des nanoparticules de silice dispersées dans de la fibroïne de soie,
dans laquelle le rapport de l'épaisseur de la couche de base à l'épaisseur de la couche bioactive est dans une plage de rapports de 5: 1 à 15: I, et
dans laquelle le rapport pondéral des nanoparticules de silice : de la fibroïne de soie : du chitosane est dans une plage de à 0.1:1:1 à 10:20:20.

2. La composition telle que revendiquée dans la revendication 1, dans laquelle les nanoparticules sont dans la plage de tailles de 10 à 1000 nm.

3. La composition telle que revendiquée dans la revendication 1, dans laquelle la composition est une composition hémostatique.

4. La composition telle que revendiquée dans l'une quelconque des revendications 1 à 3, dans laquelle la composition est sous une forme choisie parmi une mousse, un bandage, une gaze, un pansement adhésif ou de la charpie.

5. Un procédé de préparation de la composition telle que revendiquée dans la revendication I, le procédé comprenant :
a) la mise en contact de chitosane dans un solvant et la congélation pour obtenir une couche de base ;
b) l'obtention d'une première solution de fibroïne de soie dans de l'eau ;
c) la dispersion de nanoparticules de silice dans la première solution pour obtenir une seconde solution ;
d) le versement de la seconde solution sur la couche de base et la congélation pour obtenir une couche bioactive sur la couche de base, et
e) la lyophilisation de la couche bioactive sur la couche de base obtenue à l'étape (d) pour obtenir une composition stratifiée.

6. Le procédé tel que revendiqué dans la revendication 5, dans lequel le solvant est choisi parmi l'acide acétique, l'eau ou leurs combinaisons et les nanoparticules de silice sont dispersées dans de l'eau avant d'être dispersées dans la première solution.

7. Le procédé tel que revendiqué dans la revendication 5, dans lequel la congélation est mise en œuvre à une température dans une plage de -196 °C à -4 °C à la pression atmosphérique pendant une durée dans une plage de 0.1 à 15 heures ; et la lyophilisation est mise en œuvre à une température dans une plage de -90 °C à 0 °C pendant une durée dans une plage de 12 à 40 heures à une pression dans une plage de 0.1 à 1 mbar.

8. Le procédé tel que revendiqué dans la revendication 5, dans lequel la composition stratifiée est soumise à une compression ; et la compression est mise en œuvre en présence d'une presse hydraulique ou d'une presse à rouleaux à une température dans la plage de 20 °C à 40 °C.

9. Un article hémostatique comprenant la composition telle que revendiquée dans la revendication 1.

10. L'article hémostatique tel que revendiqué dans la revendication 9, dans lequel l'article hémostatique est sous une forme choisie parmi une mousse, un bandage, une gaze, un pansement adhésif ou de la charpie.

11. La composition telle que revendiquée dans la revendication 1 pour une utilisation dans l'inhibition de la perte de sang provenant d'un site hémorragique.

12. L'article hémostatique tel que revendiqué dans la revendication 9, pour une utilisation dans l'inhibition de la perte de sang provenant d'un site hémorragique.
